# NOUVEAU FASCICULE DE BREVET EUROPEEN

(11) **EP 1 047 378 B2**
(45) Date de publication et mention de la décision concernant l'opposition: **14.01.2009**
(45) Mention de la délivrance du brevet: 14.11.2001
(21) Numéro de dépôt: 98962529.8
(22) Date de dépôt: 21.12.1998
(51) Int. Cl.: A61K 8/19, A61K 8/41, A61K 8/44, A61K 8/66, A61Q 5/10

(54) **COMPOSITION TINCTORIALE CONTENANT UNE LACCASE ET PROCEDE DE TEINTURE DES FIBRES KERATINIQUES**
LACCASE ENTHALTENDES FÄRBEMITTEL UND VERFAHREN ZUR FÄRBUNG VON KERATINFASERN MIT DIESEM MITTEL
DYEING COMPOSITION CONTAINING A LACCASE AND KERATINOUS FIBRE DYEING METHOD

(30) Priorité: 13.01.1998 FR 9800250
(43) Date de publication de la demande: 02.11.2000
(73) Titulaire: L'ORÉAL, 75008 Paris (FR)
(72) Inventeur: LANG, Gérard, F-95390 Saint Prix (FR); COTTERET, Jean, F-78480 Verneuil/Seine (FR)
(74) Mandataire: Dossmann, Gérard
(86) Numéro de dépôt international: PCT/FR1998/002805
(87) Numéro de publication internationale: WO 1999/036036

(56) Documents cités:
- EP-A- 0 504 005
- WO-A-97/19999
- DE-A- 4 440 955
- FR-A- 2 694 018
- FR-A- 2 740 035

## Description

La présente invention a trait à une composition de teinture par oxydation des fibres kératiniques comprenant au moins une enzyme de type laccase, au moins un colorant d'oxydation et au moins un agent alcalinisant particulier ainsi que les procédés de teinture des fibres kératiniques notamment des cheveux humains mettant en oeuvre cette composition.

Il est connu de teindre les fibres kératiniques et en particulier les cheveux humains avec des compositions tinctoriales contenant des précurseurs de coloration d'oxydation, en particulier des ortho ou paraphénylènediamines, des ortho ou paraaminophénols, dès bases hétérocycliques, appelés généralement bases d'oxydation. Les précurseurs de coloration d'oxydation, ou bases d'oxydation, sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques

La variété des molécules mises en jeu au niveau des bases d'oxydation et des coupleurs, permet l'obtention d'une riche palette de couleurs.

La coloration dite "permanente" obtenue grâce à ces bases d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents exterieurs (lumière intemperies, lavage ondulation permanente, transpiration frottements)

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé dans le brevet US 3251742, les demandes de brevet FR-A-2 112 549, FR-A-2 694 018, EP-A-0 504 005, WO95/07988, WO95/33836, WO95/33837, WO96/00290, WO97/19998 et WO97/19999 de teindre les fibres kératiniques avec des compositions comprenant au moins un colorant d'oxydation en association avec des enzymes du type laccase ; lesdites compositions étant mises en contact avec l'oxygène de l'air. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes à la fois sur le plan de l'homogénéité de la couleur répartie le long de la fibre (« unisson »), sur le plan de la chromaticité (luminosité) et de la puissance tinctoriale.

La présente invention a pour but de résoudre les problèmes évoqués ci-dessus.

La Demanderesse a découvert de façon surprenante de nouvelles compositions contenant au moins comme système oxydant une enzyme du type laccase et au moins un agent alcalinisant particulier que l'on définira plus en détail ci-dessous, pouvant constituées en présence de colorants d'oxydation, des formulations de teinture prêtes à l'emploi conduisant à des colorations plus homogènes, plus puissantes et plus chromatiques sans engendrer de dégradation significative, ni de décoloration des fibres kératiniques, peu sélectives et résistant bien aux diverses agressions que peuvent subir les cheveux

Ces découvertes sont à la base de la présente invention.

La présente invention a donc pour premier objet une composition prête à l'emploi, destinée à la coloration par oxydation des fibres kératiniques, en particulier des fibres kératiniques humaines et plus particulièrement les cheveux humains, comprenant dans un support approprié pour la teinture des fibres kératiniques :
- **(a)** au moins une enzyme du type laccase ;
- **(b)** au moins un agent alcalinisant choisi dans le groupe formé par :
   **(i)** un acide aminé basique ;
   **(ii)** un composé de formule **(A)** suivante :
      X(OH)ₙ dans laquelle, X représente K, lorsque n=1 ;
   **(iii)** la monoisopropanolamine
- **(c)** au moins un colorant d'oxydation à l'exception des colorants indoliques autooxydables.

La ou les laccases utilisées dans la composition tinctoriale prête à l'emploi conforme à l'invention peuvent notamment être choisies parmi les laccases d'origine végétale, d'origine animale, d'origine fongique (levures, moisissures, champignons) ou d'origine bactérienne, les organismes d'origine pouvant être mono ou pluricellulaires. Elles peuvent être obtenues par biotechnologie.

Parmi les laccases d'origine végétale utilisables selon l'invention , on peut citer les laccases produites par des végétaux effectuant la synthèse chlorophyllienne telles qu'indiquées dans la demande FR-A-2 694 018 comme celles que l'on retrouve dans les extraits des Anacardiacées tels que par exemple les extraits de Magnifera indica, Schinus molle ou Pleiogynium timoriense. dans les extraits des Podocarpacées. de Rosmarinus off, de Soianum tuberosum d'Iris sp. de Coffea sp. de Daucus carrota, de Vinca miner Persea americana. Catharenthus roseus, Musa sp., Malus pumila, Gingko biloba, Monotropa hypopithys (sucepin), Aesculus sp., Acer pseudoplatanus, Prunus persica, Pistacia palaestina.

Parmi les laccases d'origine fongique éventuellement obtenues par biotechnologie utilisables selon l'invention, on peut citer la ou les laccases issues de Polyporus versicolor, de Rhizoctonia praticola et de Rhus vernicifera comme indiquées dans les demandes FR-A-2 112 549 et EP-A-504005 ; celles décrites dans les demandes de brevet WO95/07988, WO95/33836, WO95/33837, WO96/00290, WO97/19998 et WO97/19999, comme par exemple celles issues de Scytalidium, de Polyporus pinsitus, de Myceliophtora thermophila, de Rhizoctonia solani, de Pyricularia orizae, ou leurs variantes. On peut aussi citer celles issues de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens et de leurs variantes.

On choisira plus préférentiellement les laccases d'origine fongique éventuellement obtenues par biotechnologie.

L'activité enzymatique des laccases de l'invention ayant la syringaldazine parmi leurs substrats peut être définie à partir de l'oxydation de la syringaldazine en condition aérobie. L'unité lacu correspond à la quantité d'enzyme catalysant la conversion de immole de syringaldazine par minute à pH 5.5 à 30°C. L'unité u correspond à la quantité d'enzyme produisant un delta d'absorbance à 530 nm de 0,001 par minute en utilisant la syringaldazine comme substrat, à 30°C et à pH 6,5.
L'activité enzymatique des laccases de l'invention peut aussi être définie à partir de l'oxydation de la paraphénylènediamine. L'unité ulac correspond à la quantité d'enzyme produisant un delta d'absorbance à 496,5nm de 0,001 par minute en utilisant la paraphénylènediamine comme substrat (64 mM) à 30°C et à pH 5.
Selon l'invention, on préfère déterminer l'activité enzymatique en unités ulac.
Les quantités de laccase utilisées dans les compositions de l'invention varieront en fonction de la nature de la laccase choisie. De façon préférentielle, elles varieront de 0,5 à 2000 lacu, ou de 1000 à 4.10⁷ unités u. ou de 20 à 2.10⁶ unités uiac pour 100g de composition.

Au sens de la présente invention, i.e. dans le texte qui précède et qui suit, on définit, par "acide aminé basique", soit (i) un acide aminé présentant en plus de la fonction amine positionnée en α du groupement carboxyle, un groupement cationique (ou basique) supplémentaire; soit (ii) un acide aminé présentant une chaîne latérale (hydrophile) cationique (ou basique); soit (iii) un acide aminé portant une chaîne latérale constituée d'une base azotée. Ces définitions sont généralement connues et publiées dans les ouvrages de biochimie générale tels que J.H. WEIL (1983) pages 5 et suivantes, Lubert STRYER (1995) page 22 A. LEHNINGER (1993) pages 115-116. DE BOECK-WESMAEL (1994) pages 57-59.

Les acides aminés basiques conformes à l'invention sont choisis de préférence parmi ceux répondant à la formule (D) suivante 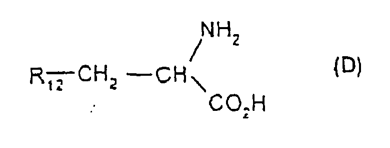 où R₁₂ désigne un groupe choisi parmi

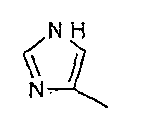

-(CH₂)₃NH₂ ;

-(CH₂)₂NH₂

-(CH₂)₂NHCONH₂ ;

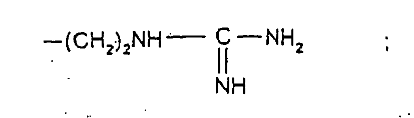

Les composés correspondants à la formule (D) sont l'histidine, la lysine, l'ornithine, la citrulline, l'arginine.

Dans les composés de formule (A) ou (B), selon l'invention, les radicaux alkyles peuvent être linéaires ou ramifiés et les radicaux polyhydroxyalkyle désignent des radicaux comportant de 2 à 6 groupes hydroxy et de préférence de 2 à 4.

Les compositions conformes à l'invention renferment les agents alcalinisants particuliers définis ci-dessus à des teneurs pondérales qui peuvent aller de 0.001% à 20 %, de préférence de 0.01 % à 5 % et encore plus préférentiellement de 0.05 % à 3%, par rapport au poids total de la composition.

La nature du ou des colorants d'oxydation utilisés dans la composition tinctoriale prête à l'emploi n'est pas critique. Ils sont choisis parmi les bases d'oxydation et/ou les coupleurs.

Les bases d'oxydation peuvent notamment être choisies parmi les ortho-paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (I) suivante et leurs sels d'addition avec un acide : 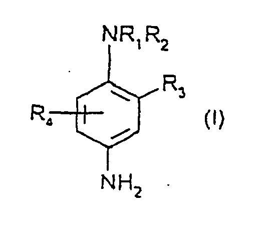 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C4), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté :
- R₃ représente un atome d'hydrogène, un atome d'halogène tel qu'un atome de chlore. de brome, d'iode ou de fluor, un radical alkyle en C₁-C₄, monohydroxyalkyle en C₁-C₄, hydroxyalcoxy en C₁-C₄, acétylaminoaicoxy en C₁-C₄, mésylaminoalcoxy en C₁-C₄ ou carbamoylaminoalcoxy en C₁-C₄,
- R₄ représente un arôme d'hydrogène d'halogène ou un radical alkyle en C₁-C₄

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 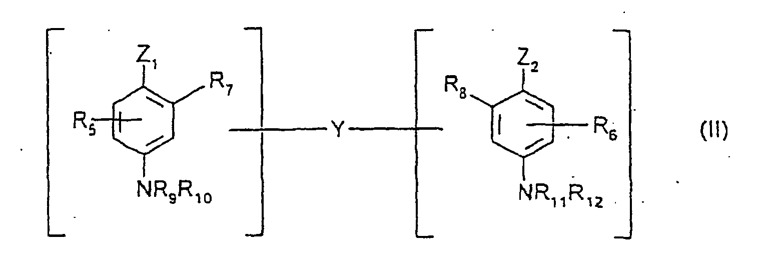 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (III) suivante, et leurs sels d'addition avec un acide : 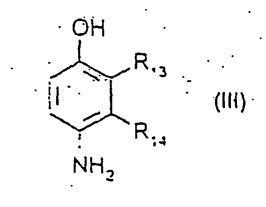 dans laquelle
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-métnoxy pyridine, la 3 4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3.5-diamino 1-méthyl 4-méthylamino pyrazole, le 3,5-diamino 4-(β-hydroxyéthyl)amino 1-méthyl pyrazole, et leurs sels d'addition avec un acide

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères. lorsqu'il existe un équilibre tautomérique : 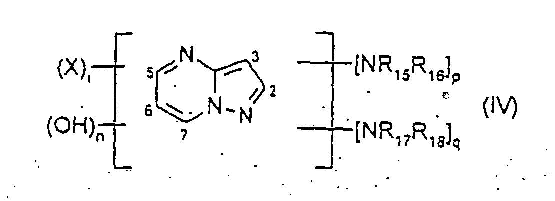 dans laquelle :
- R₁₅, R₁₆, R₁₇ et R₁₈, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que
- la somme p + q est différente de 0.
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7) ;

Lorsque les pyrazolo-[1,5-a]-pyrimidines de formule (IV) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

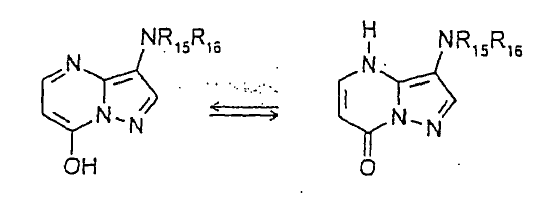

Parmi les pyrazolo-[1,5-a]-pyrimidines de formule (IV) ci-dessus on peut notamment citer :
- la pyrazolo-[1,5-a]-pyrimidine-37-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2.6-diméthyl pyrazolo-[1,5-a]-pyrimidine-3,7-diamine ;
- la 2, 5, N 7, N 7-tetraméthyl pyrazolo-[1 5-a]-pyrimidine-3,7-diamine ;
et leurs sels d'addition et leurs formes tautomères, lorsqu'il existe un équilibre tautomérique.

Les pyrazolo-[1,5-a]-pyrimidines de formule (IV) ci-dessus peuvent être préparées par cyclisation à partir d'un aminopyrazole selon les synthèses décrites dans les références suivantes :
- EP 628559 BEIERSDORF-LILLY
- R. Vishdu, H. Navedul, Indian J. Chem., 34b (6), 514, 1995,
- N.S. Ibrahim, K.U. Sadek, F.A. Abdel-Al, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (IV) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Almera, J. Elguero. J. Heterocyclic Chem., 11(3), 423, 1974.
- K. Saito, I. Hori, M. Higarashi, H. Midorikawa, Bull. Chem. Soc. Japan, 47(2), 476, 1974.

La ou les bases d'oxydation représentent de préférence de 0,0005 à 12 % en poids environ du poids total de la composition tinctoriaie conforme à l'invention, et encore plus préférentiellement de 0.005 à 6 % en poids environ de ce poids.

Le ou les coupleurs pouvant être utilisés dans la composition tinctoriale prête à l'emploi conforme à l'invention sont ceux classiquement utilisés dans les compositions de teinture d'oxydation, c'est-à-dire des meta-phénylènediamines. des méta-aminophénols, des métadiphénols, des coupleurs hétérocycliques, et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, le 2.6-diméthyl-pyrazolo-[1,5-b]-1,2,4-triazole, le 2,6-diméthyl-[3,2-c]-12,4-triazole, le 6-méthyl-pyrazolo-[1,5-a]-benzimidazole, et leurs sels d'addition avec un acide.

Ces coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

La composition tinctoriale de l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques.

La composition tinctoriale prête à l'emploi conforme à l'inventicn peut également renfermer divers adjuvants utilisés classiquement dans les compositions pour la teinture des cheveux, tels que des agents tensio-actifs anioniques, cationiques. non-ioniques, amphotères, zwittérioniques ou leurs mélanges, des polymères, des agents épaississsants, des agents antioxydants, des enzymes différentes des laccases utilisées conformément à l'invention telles que par exemple des peroxydases ou des oxydo-réductases à 2 électrons, des agents de pénétration, des agents séquestrants, des parfums, des agents dispersants, des agents filmogènes, des agents filtrants, des vitamines, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le ou les colorants d'oxydation et la ou les laccases sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince. on lave éventueliement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins un colorant d'oxydation tel que défini précédemment et, d'autre part, une composition (B) renfermant, dans un milieu approprié pour la teinture, au moins une enzyme de type laccase et au moins un agent alcalinisant particulier tels que définis ci-dessus, puis à procéder à leur mélange au moment de l'emploi avant d'appliquer ce mélange sur les fibres kératiniques.

Selon une forme de réalisation particulière de l'invention, l'agent alcalinisant peut être incorporé dans la composition (A).

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Le milieu approprié pour les fibres kératiniques (ou support) des compositions tinctoriales conformes à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ainsi que les alcools aromatiques comme l'alcool benzylique, les produits analogues et leurs mélanges

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH des compositions tinctoriales conformes à l'invention est choisi de telle manière que l'activité enzymatique de la laccase ne soit pas altérée. Il varie généralement de 6 à 11 environ, et plus préférentiellement de 6 à 9 environ.

Des exemples concrets illustrant l'invention vont maintenant être donnés.

Dans ce qui suit ou ce qui précède, sauf mention contraire, les pourcentages sont exprimés en poids.

Les exemples suivants illustrent l'invention sans présenter un caractère limitatif.

### EXEMPLE 1 : Composition de teinture

On a préparé la composition tinctoriale prête à l'emploi suivante (teneurs en grammes) :
- Laccase issue de la Rhus vernicifera à 180 unités/mg unités / mg, commercialisée par la société SIGMA. 1,8 g
- Alkyl (C₈-C₁₀) polyglucoside en solution aqueuse à 60 % de matière active (M.A.) vendu sous la dénomination ORAMIX CG110 par la société SEPPIC 8.0 g
- Paraphénylènediamine 0,254 g
- Dichlorhydrate de 2.4-diaminophénoxyéthanol 0.260 g
- Arginine qs pH 6.5
- Eau déminéralisée qsp 100 g

Cette composition tinctoriale prête à l'emploi a été appliquée sur des mèches de cheveux gris naturels à 90 % de blancs pendant 40 minutes à 30°C. Les cheveux ont ensuite été rincés, lavés avec un shampooing standard, puis séchés.

On obtient des mèches de cheveux teintes en gris bleuté.

Dans cet exemple, 1,8 g de laccase issue de Rhus vernicifera à 180 unités/mg. peut être remplacé par 1g de laccase issue de Pyricularia Orizae à 100 unités/mg vendue par la société I.C.N.

### EXEMPLE 2 : Composition de teinture

On a préparé la composition tinctoriale prête à l'emploi suivante (teneurs en grammes) :
- Laccase issue de la Rhus vernicifera à 180 unités/mg unités / mg, commercialisée par la société SIGMA. 1,8 g
- Alkyl (C₈-C₁₀) polyglucoside en solution aqueuse à 60 % de matière active (M.A.) vendu sous la dénomination ORAMIX CG110 par la société SEPPIC 8,0 g
- Paraphénylènediamine 0,254 g
- Dichlorhydrate de 2,4-diaminophénoxyéthanol 0,260 g
- Ethanol 20,0 g
- Citrulline qs pH 8,0
- Eau déminéralisée qsp 100 g

Cette composition tinctoriale prête à l'emploi a été appliquée sur des mèches de cheveux gris naturels à 90 % de blancs pendant 40 minutes à 30°C. Les cheveux ont ensuite été rincés, lavés avec un shampooing standard, puis séchés.

On obtient des mèches de cheveux teintes en gris bleuté.

Dans cet exemple, 1,8 g de laccase issue de Rhus vernicifera à 180 unités/mg peut être remplacé par 1g de laccase issue de Pyricularia Orizae à 100 unités/mg vendue par la société I.C.N.

## Revendications

1. Composition prête à l'emploi pour la teinture par oxydation des fibres kératiniques, en particulier des fibres kératiniques humaines et plus particulièrement les cheveux humains, comprenant dans un support approprié pour la teinture des fibres kératiniques:
- (a) au moins une enzyme du type laccase ;
- (b) au moins un agent alcalinisant choisi dans le groupe formé par:
(i) un acide aminé basique;
(ii) un composé de formule (A) suivante :
X(OH)ₙ dans laquelle, X représente K, lorsque n=1 ;
(iii) la monoisopropanolamine
- (c) au moins un colorant d'oxydation à l'exception des colorants indoliques autooxydables.

2. Composition selon la revendication 1, **caractérisée par le fait que** la ou les laccases sont choisies parmi les laccases d'origine vegétale, d'origine animale, d'origine fongique, d'origine bactérienne, ou obtenues par biotechnologie.

3. Composition selon l'une quelconque des revendications 1 à 2, où les laccases sont choisies parmi celles produites par des végétaux effectuant la synthèse chlorophyllienne.

4. Composition selon la revendication 3, où les laccases sont choisies parmi celles extraites des Anacardiacées ou des Podocarpacées de Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin) Aesculus sp., Acer pseudoplatanus, Prunus persica, Pistacia palaestina.

5. Composition selon la revendication 2, où les laccases sont choisies parmi celles issues de Pyricularia orizae, de Polyporus versicolor, de Rhizoctonia praticola, de Rhus vernicifera, de Scytalidium, de Polyporus pinsitus, de Myceliophtora thermophila, de Rhizoctonia solani, de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, ainsi que leurs variantes.

6. Composition selon l'une quelconque des revendications 1 à 5, **caractérisée par le fait que** la ou les laccases sont présentes dans des quantités allant de 0,5 à 2000 lacu, ou de 1000 à 4.10⁷ unités u, ou de 20 à 2.10⁵ unités ulac, pour 100g de composition.

7. Composition selon l'une quelconque des revendications 1 à 6, **caractérisée par le fait que** les acides aminés basiques répondent à la formule (D) suivante: 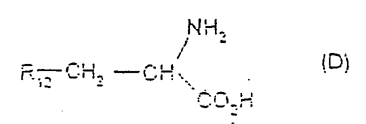 où R₁₂ désigne un groupe choisi parmi : 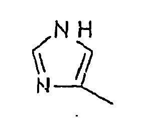
-(CH₂)₃NH₂ ;
-(CH₂)₂NH₂
-(CH₂)₂NHCONH₂ ;

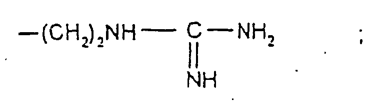

8. Composition selon l'une quelconque des revendications précédentes, **caractérisée par le fait que** les agents alcalinisants sont utilisés à des teneurs pondérales allant de 0.001% à 20 %, de préférence de 0,01 % à 5 % et encore plus préférentiellement de 0,05 % à 3%, par rapport au poids total de la composition.

9. Composition selon l'une quelconque des revendications précédentes, **caractérisée par le fait que** les colorants d'oxydation sont des bases d'oxydation choisies parmi les ortho- ou para- phénylènediamines, les bis-phénylalkylénediamines, les ortho- ou para- aminophénols, et les bases hétérocyliques, ainsi que les sels d'addition de ces composés avec un acide.

10. Composition selon la revendication 9, **caractérisée par le fait que** les bases d'oxydation sont présents dans des concentrations allant de 0,0005 à 12% en poids par rapport au poids total de la composition.

11. Composition selon la revendication 9, **caractérisée par le fait que** les colorants d'oxydation sont des coupleurs choisis parmi les métaphénylènediamines, les métaminophénols, les métadiphénols, les coupleurs hétérocycliques, et les sels d'addition de ces composés avec un acide.

12. Composition selon la revendication 11, **caractérisée par le fait que** les coupleurs sont présents dans des concentrations allant de 0.0001 à 10% en poids par rapport au poids total de la composition.

13. Composition selon l'une quelconque des revendications 9 à 12, **caractérisée par le fait que** les sels d'addition avec un acide des colorants d'oxydation sont choisis parmi les chlorhydrates, les bromhydrates, les sulfates, les tartrates, les lactates et les acétates.

14. Composition selon l'une quelconque des revendications 1 à 13, **caractérisée par le fait qu'**elle contient en outre des colorants directs.

15. Composition selon l'une quelconque des revendications 1 à 14, **caractérisée par le fait que** le milieu approprié pour les fibres keratiniques (ou support) est constitué par de l'eau ou par un mélange d'eau et d'au moins un solvant organique.

16. Composition selon la revendication 15, **caractérisée par le fait que** les solvants organiques peuvent être présents dans des proportions de préférence allant 1 à 40 % en poids environ par rapport au poids total de la composition, et encore plus préférentiellement allant de 5 à 30 % en poids environ.

17. Composition selon l'une quelconque des revendications 1 à 16, **caractérisée par** le lait que le pH varie de 6 à 11 environ, et de préférence de 6 à 9 environ.

18. Composition, selon l'une quelconque des revendications 1 à 17, **caractérisée par le fait qu'**elle contient en plus au moins un adjuvant cosmétique utilisé classiquement dans les compositions pour la teinture des cheveux choisi dans le groupe constitué par des agents tensio-actifs, des polymères, des agents épaississsants, des agents antioxydants, des enzymes différentes des laccases, des agents de pénétration, des agents séquestrants, des parfums, des agents dispersants, des agents filmogènes, des agents filtrants, des vitamines, des agents conservateurs, des agents opacifiants.

19. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications 1 à 18, pendant un temps suffisant pour développer la coloration désirée.

20. Procédé selon la revendication 19, **caractérisé par le fait qu'**il comporte une étape préliminaire consistant à stocker sous forme séparée, d'une part, une composition (A) comprenant, dans un milieu approprié pour la teinture, au moins un colorant d'oxydation tel que défini dans l'une quelconque des revendications 1 et 10 à 14 et d'autre part, une composition (B) renfermant, dans un milieu approprié pour les fibres kératiniques, au moins une enzyme du type laccase telle que définie dans l'une quelconque des revendications 1 à 6 puis à procéder à leur mélange au moment de l'emploi avant d'appliquer ce mélange sur les fibres kératiniques; la composition (A) ou la composition (B) contenant l'agent alcalinisant tel que défini dans les revendications 1 et 7 et 8.

21. Dispositif à plusieurs compartiments ou "kit" de teinture, **caractérisé par le fait qu'**il comporte un premier compartiment renfermant la composition (A) telle que définie dans la revendication 20 et un second compartiment renfermant la composition (B) telle que définie dans la revendication 20.

## Claims

1. Ready-to-use composition for the oxidation dyeing of keratinous fibres, in particular human keratinous fibres and more particularly human hair, comprising, in a carrier appropriate for dyeing keratinous fibres:
- (a) at least one enzyme of the laccase type;
- (b) at least one alkalinizing agent chosen from the group consisting of:
(i) a basic amino acid;
(ii) a compound of the following formula (A):
X(OH)ₙ in which X represents K when n=1;
(iii) monoisopropanolamine;
- (c) at least one oxidation dye with the exception of autooxidizable indole dyes.

2. Composition according to Claim 1, **characterized in that** the laccase(s) are chosen from laccases of plant origin, animal origin, fungal origin, bacterial origin or are obtained by biotechnology.

3. Composition according to either of Claims 1 to 2, where the laccases are chosen from those produced by plants performing chlorophyll synthesis.

4. Composition according to Claim 3, where the laccases are chosen from those extracted from Anacardiaceae or Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Indian pipe), Aesculus sp., Acer pseudoplatanus, Prunus persica, Pistacia palaestina.

5. Composition according to Claim 2, where the laccases are chosen from those derived from Pyricularia orizae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitius squalens and variants thereof.

6. Composition according to any one of Claims 1 to 5, **characterized in that** the laccase(s) are provided in quantities ranging from 0.5 to 2000 lacu, or from 1000 to 4x10⁷, or from 2x10⁶ lacu units, per 100 g of composition.

7. Composition according to any one of Claims 1 to 6, **characterized in that** the basic amino acids correspond to the following formula (D): 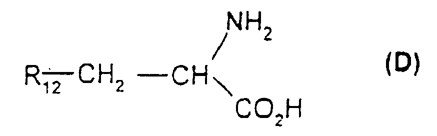 where R₁₂ denotes a group chosen from: 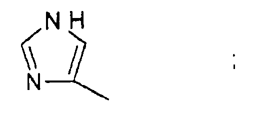
-(CH₂)₃NH₂ ;
-(CH₂)₂NH₂
-(CH₂)₂NHCONH₂ ;

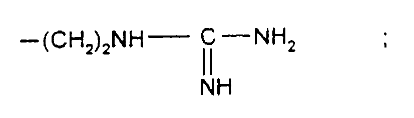

8. Composition according to any one of the preceding claims, **characterized in that** the alkalinizing agents are used in contents by weight ranging from 0.001% to 20%, preferably from 0.01% to 5% and still more preferably from 0.05% to 3%, relative to the total weight of the composition.

9. Composition according to any one of the preceding claims, **characterized in that** the oxidation dyes are oxidation bases chosen from ortho- or paraphenylenediamines, bisphenylalkylenediamines, ortho- or para-aminophenols, and heterocyclic bases, as well as the addition salts of these compounds with an acid.

10. Composition according to Claim 9, **characterized in that** the oxidation bases are present in concentrations ranging from 0.0005 to 12% by weight relative to the total weight of the composition.

11. Composition according to Claim 9, **characterized in that** the oxidation dyes are couplers chosen from meta-phenylenediamines, meta-aminophenols, meta-diphenols, heterocyclic couplers, and the addition salts of these compounds with an acid.

12. Composition according to Claim 11, **characterized in that** the couplers are present in concentrations ranging from 0.0001 to 10% by weight relative to the total weight of the composition.

13. Composition according to any one of Claims 9 to 12, **characterized in that** the addition salts with an acid of the oxidation dyes are chosen from hydrochlorides, hydrobromides, sulphates, tartrates, lactates and acetates.

14. Composition according to any one of Claims 1 to 13, **characterized in that** it contains, in addition, direct dyes.

15. Composition according to any one of Claims 1 to 14, **characterized in that** the medium appropriate for keratinous fibres (or carrier) consists of water or of a mixture of water and of at least one organic solvent.

16. Composition according to Claim 15, **characterized in that** the organic solvents may be present in proportions preferably ranging from 1 to 40% by weight approximately relative to the total weight of the composition, and still more preferably ranging from 5 to 30% by weight approximately.

17. Composition according to any one of Claims 1 to 16, **characterized in that** the pH varies from 6 to 11 approximately, and preferably from 6 to 9 approximately.

18. Composition according to any one of Claims 1 to 17, **characterized in that** it contains, in addition, at least one cosmetic adjuvant conventionally used in hair dyeing compositions, chosen from the group consisting of surfactants, polymers, thickeners, antioxidants, enzymes different from the lacasses, penetrating agents, sequestering agents, perfumes, dispersing agents, film-forming agents, screening agents, vitamins, preservatives or opacifying agents.

19. Method of dyeing keratinous fibres, and in particular human keratinous fibres such as hair, **characterized in that** at least one ready-to-use dyeing composition as defined in any one of Claims 1 to 18 is applied to the said fibres for a sufficient time to develop the desired colour.

20. Method according to Claim 19, **characterized in that** it comprises a preliminary step consisting in storing in a separate form, on the one hand, a composition (A) comprising, in a medium appropriate for dyeing, at least one oxidation dye as defined in any one of Claims 1 and 10 to 14 and, on the other hand, a composition (B) containing, in a medium appropriate for keratinous fibres, at least one enzyme of the laccase type as defined in any one of Claims 1 to 6, and then in mixing them at the time of use before applying this mixture to the keratinous fibres; the composition (A) or the composition (B) containing the alkalinizing agent as defined in Claims 1 and 7 and 8.

21. Multicompartment device or dyeing "kit", **characterized in that** it comprises a first compartment containing the composition (A) as defined in Claim 20 and a second compartment containing the composition (B) as defined in Claim 20.

## Patentansprüche

1. Gebrauchsfertige Zusammensetzung zum oxidativen Färben von Keratinfasern, insbesondere menschlichen Keratinfasern und besonders zum Färben menschlicher Haare, die in einem zum Färben von Keratinfasern geeigneten Träger enthält:
(a) mindestens ein Enzym vom Laccase-Typ;
(b) mindestens ein Alkalisierungsmittel, das ausgewählt ist unter:
(i) basischen Aminosäuren;
(ii) Verbindungen der folgenden Formel (A):
X(OH)ₙ, wobei X K bedeutet, wenn n = 1;
(iii) Monoisopropanolamin;
(c) mindestens einen Oxidationsfarbstoff, wobei selbstoxiderfähige Indolfarbstoffe ausgenommen sind.

2. Zusammensetzung nach Anspruch 1, **dadurch gekennzeichnet, dass** die Laccase(n) unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt sind.

3. Zusammensetzung nach einem der Ansprüche 1 oder 2, wobei die Laccasen unter den Laccasen ausgewählt sind, die von Pflanzen gebildet werden, die Chloropyll synthetisieren.

4. Zusammensetzung nach Anspruch 3, wobei die Laccasen unter den Laccasen ausgewählt sind, die aus Anacardiaceae oder Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica oder Pistacia palaestina extrahiert wurden.

5. Zusammensetzung nach Anspruch 2, wobei die Laccasen unter den Laccasen ausgewählt sind, die von Pyricularia oryzae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

6. Zusammensetzung nach einem der Ansprüche 1 bis 5, **dadurch gekennzeichnet, dass** die Laccase(n) in einem Mengenanteil im Bereich von 0,5 bis 2000 lacu oder 1000 bis 4.10⁷ Einheiten u oder 20 bis 2.10⁶ Einheiten ulac pro 100 g Zusammensetzung vorliegen.

7. Zusammensetzung nach einem der Ansprüche 1 bis 6, **dadurch gekennzeichnet, dass** die basischen Aminosäuren der folgenden Formel (D) entsprechen: 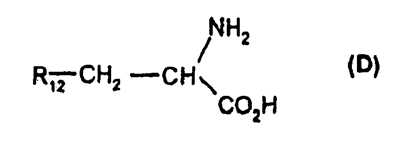 worin R₁₂ eine Gruppe bedeutet, die ausgewählt ist unter: 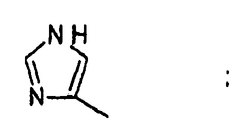
-(CH₂)₃NH₂ ;
-(CH₂)₂NH₂
-(CH₂)₂NHCONH₂ ;

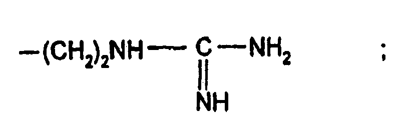

8. Zusammensetzung nach einem der vorhergehenden Ansprüche, **dadurch gekennzeichnet, dass** die Alkalisierungsmittel in Gewichtsanteilen von 0,001 bis 20 %, vorzugsweise 0,01 bis 5 % und noch bevorzugter 0,05 bis 3 %, bezogen auf das Gesamtgewicht der Zusammensetzung, verwendet werden.

9. Zusammensetzung nach einem der vorhergehenden Ansprüche, **dadurch gekennzeichnet, dass** die Oxidationsfarbstoffe Oxidationsbasen sind, die unter den o- oder p-Phenylendiaminen, Bisphenylalkylendiaminen, o- oder p-Aminophenolen und den heterocyclischen Basen sowie den Additionsverbindungen dieser Verbindungen mit einer Säure ausgewählt sind.

10. Zusammensetzung nach Anspruch 9, **dadurch gekennzeichnet, dass** die Oxidationsbasen in Konzentrationen von 0,0005 bis 12 Gew.-%, bezogen auf das Gesamtgewicht der Zusammensetzung, vorliegen.

11. Zusammensetzung nach Anspruch 9, **dadurch gekennzeichnet, dass** die Oxidationsfarbstoffe Kuppler sind, die unter den m-Phenylendiaminen, m-Aminophenolen, m-Dihydroxybenzolen und heterocyclischen Kupplern und deren Additionssalzen mit einer Säure ausgewählt sind.

12. Zusammensetzung nach Anspruch 11, **dadurch gekennzeichnet, dass** die Kuppler in Konzentrationen von 0,0001 bis 10 Gew.-%, bezogen auf das Gesamtgewicht der Zusammensetzung, vorliegen.

13. Zusammensetzung nach einem der Ansprüche 9 bis 12, **dadurch gekennzeichnet, dass** die Additionssalze mit einer Säure unter den Hydrochloriden, Hydrobromiden, Sulfaten, Tartraten, Lactaten und Acetaten ausgewählt sind.

14. Zusammensetzung nach einem der Ansprüche 1 bis 13, **dadurch gekennzeichnet, dass** sie ferner Direktfarbstoffe enthält.

15. Zusammensetzung nach einem der Ansprüche 1 bis 14, **dadurch gekennzeichnet, dass** das für Keratinfasern geeignete Medium (oder der Träger) aus Wasser oder einem Gemisch von Wasser und mindestens einem organischen Lösungsmittel besteht.

16. Zusammensetzung nach Anspruch 15, **dadurch gekennzeichnet, dass** die organischen Lösungsmittel vorzugsweise in Mengenanteilen von etwa 1 bis 40 Gew.-%, bezogen auf das Gesamtgewicht der Zusammensetzung, und noch bevorzugter 5 bis 30 Gew.-% vorliegen können.

17. Zusammensetzung nach einem der Ansprüche 1 bis 16, **dadurch gekennzeichnet, dass** sie einen pH-Wert im Bereich von etwa 6 bis 11 und vorzugsweise etwa 6 bis 9 aufweist.

18. Zusammensetzung nach einem der Ansprüche 1 bis 17, **dadurch gekennzeichnet, dass** sie ferner mindestens einen herkömmlich in Zusammensetzungen zum Färben verwendeten kosmetischen Zusatzstoff enthält, der unter den grenzflächenaktiven Stoffen, Polymeren, Verdickungsmitteln, Antioxidantien, Enzymen, die von Laccasen verschieden sind, Penetrationsmitteln, Maskierungsmitteln, Parfums, Dispergiermitteln, Filmbildnern, Filtern, Vitaminen, Konservierungsmitteln und Trübungsmitteln ausgewählt sind.

19. Verfahren zum Färben von Keratinfasern und insbesondere menschlichen Keratinfasern, wie dem Haar, **dadurch gekennzeichnet, dass** auf die Fasern während einer Zeitspanne, die zur Bildung einer Färbung ausreichend ist, mindestens eine gebrauchsfertige Farbmittelzusammensetzung nach einem der Ansprüche 1 bis 18 aufgebracht wird.

20. Verfahren nach Anspruch 19, **dadurch gekennzeichnet, dass** es einen vorbereitenden Schritt umfasst, der darin besteht, einerseits eine Zusammensetzung (A), die in einem zum Färben geeigneten Medium mindestens einen Oxidationsfarbstoff nach einem der Ansprüche 1 und 10 bis 14 enthält, und andererseits eine Zusammensetzung (B) getrennt voneinander aufzubewahren, die in einem für Keratinfasern geeigneten Medium mindestens ein Enzym vom Laccase-Typ nach einem der Ansprüche 1 bis 6 enthält, und diese bei der Anwendung zu vermischen, bevor das Gemisch auf die Keratinfasern aufgebracht wird, wobei die Zusammensetzung (A) oder die Zusammensetzung (B) ein Alkalisierungsmittel nach einem der Ansprüche 1 und 7 und 8 enthält.

21. Vorrichtung mit mehreren Abteilungen oder "Kit" zum Färben, **dadurch gekennzeichnet, dass** sie eine erste Abteilung mit der in Anspruch 20 definierten Zusammensetzung (A) und eine zweite Abteilung mit der in Anspruch 20 definierten Zusammensetzung (B) enthält.
